**Europäisches Patentamt**

**European Patent Office**

**Office européen des brevets**

(11) Veröffentlichungsnummer: **0 253 255**
**A2**

# (12) EUROPÄISCHE PATENTANMELDUNG

(21) Anmeldenummer: **87109687.1**

(22) Anmeldetag: **06.07.87**

(51) Int. Cl.⁴: **G01N 33/68 , G01N 33/86**

(30) Priorität: **09.07.86 DD 292298**

(43) Veröffentlichungstag der Anmeldung:
**20.01.88 Patentblatt 88/03**

(84) Benannte Vertragsstaaten:
**AT CH DE FR GB LI**

(71) Anmelder: **VEB Arzneimittelwerk Dresden**
**Wilhelm-Pieck-Strasse 35**
**DDR-8122 Radebeul 1(DD)**

(72) Erfinder: **Eckardt, Klaus, Dr. rer. nat.**
**Erfurter Strasse 58**
**DD-6900 Jena(DD)**
Erfinder: **Stepanauskas, Marlena Dr. med.**

**verstorben(DD)**
Erfinder: **Thrum, Heinz, Dr. rer. nat.**

**verstorben(DD)**
Erfinder: **Töpfer, Gottfried, Dr. rer. nat.**
**Am Feierabendheim 26**
**DD-8909 Görlitz(DD)**
Erfinder: **Seifert, Andreas, Dr. rer. nat.**
**Theodor-Körner-Strasse 5**
**DD-8900 Görlitz(DD)**
Erfinder: **Schulze, Manfred, Dr. med.**
**Ludwigstrasse 2**
**DD-8800 Zittau(DD)**
Erfinder: **Funke, Udo, Dipl.-Chem.**
**Rietschelstrasse 20**
**DD-8800 Zittau(DD)**

(74) Vertreter: **Patentanwälte Beetz sen. - Beetz**
**jun. Timpe - Siegfried - Schmitt-Fumian**
**Steinsdorfstrasse 10**
**D-8000 München 22(DE)**

(54) **Verfahren und Testkit zum Nachweis von Fibrinmonomeren im Blut.**

(57) Die Erfindung betrifft ein Verfahren sowie eine Testkombination zum Nachweis von Fibrinmonomeren in menschlichem Blut. Bei diesem Verfahren vermischt man zunächst Venenblut in bekannter Weise mit einem Anticoagulans und versetzt das nach Zentrifugation erhaltene Blutplasma erfindungsgemäß mit einem Antibioticum der Pyrrolamidin-Reihe als Fällungsmittel, insbesondere mit Netropsin, Distamycin und/oder Anthelvencin. Nach Stehenlassen wird die Probe zentrifugiert und das sedimentierte Präzipitat in an sich bekannter Weise beurteilt.

Entsprechende Testkombinationen enthalten das Fällungsmittel sowie einen Puffer, bevorzugt HEPES.

## Verfahren und Testkit zum Nachweis von Fibrinmonomeren im Blut

Die Erfindung betrifft ein Verfahren sowie eine Testkombination zum Nachweis von Fibrinmonomeren im menschlichen Blutplasma.

Das Auftreten von Fibrinmonomeren im Blut beweist eine Aktivierung des Hämostasesystems. Sie ist eine klinisch häufig auftretende Begleiterscheinung zahlreicher Grunderkrankungen. Dabei können sich über das Stadium der Hypercoagulabilität lokal bedingt Thrombosen oder generalisiert Verbrauchscoagulopathien mit schweren Blutungsfolgen entwickeln. Ein möglichst frühzeitiger Nachweis der Gerinnungsaktivität ist hierbei die Voraussetzung für eine effektive Therapie.

Es sind bereits mehrere Nachweisprinzipien für Fibrinmonomere bekannt. Die ältesten Verfahren sind der Ethanolgelationstest nach Godal (H. Godal und U. Abilgaard, Scand. J. Haem, 3 (1966) 342) und der Protaminsulfattest nach Lipinski (B. Lipinski und K. Warowski, Thromb. Diath. Haem. 20 (1968) 44). Diese Verfahren sind leicht durchführbar, zeigen aber eine unzureichende Sensitivität und Spezifität. Ein spezifischer Test ist der Agglutinationstest mit fibrinmonomerbeladenen Erythrocyten nach Largo (R. Largo et al., Blood 47 - (1976), 991). Weiterhin wird auch der zur Diagnose der Willebrandschen Krankheit geeignete Test auf der Basis der Fällungsmittel Ristocetin-A bzw. Ristomycin-A zum Nachweis von Fibrinmonomeren angewandt (K. Watanabe und J. L. Tullis, Amer. J. Clin. Pathol. 70 (1978) 691; G. Pfliegler et al., Abstr. Int. Congr. ISH-ISBT, Budapest, Aug. 1982, p. 380). Umfangreiche Vergleichsuntersuchungen haben gezeigt, daß mit den genannten Fällungsmitteln eine unspezifische Präzipitation mit Fibrinspaltprodukten stattfindet und mit steigendem Thrombocytengehalt des zum Nachweis eingesetzten Plasmas falsche positive Reaktionen erhalten werden, so daß die Sicherheit des Tests nicht gewährleistet ist. Über diese Verfahren hinaus existieren verschiedene, an aufwendige Analysentechnik gebundene Methoden (Affinitätschromatographie, Gelfiltration).

Der Erfindung liegt die Aufgabe zugrunde, eine neuartige Testkombination zum Nachweis von Fibrinmonomeren im menschlichen Blut sowie auf dieser Basis ein kostengünstiges Verfahren zum Nachweis von Fibrinmonomeren durch Fällung anzugeben.

Die Aufgabe wird gemäß den Patentansprüchen 1 und 9 gelöst. Die abhängigen Ansprüche betreffen vorteilhafte Ausführungsformen.

Die erfindungsgemäße Testkombination ist gekennzeichnet durch Gehalt eines Antibioticums der Pyrrolamidin-Reihe als Fällungsmittel, das bevorzugt in einer Pufferlösung vorliegt. Vorzugsweise werden in der Testkombination als Pyrrolamidin-Antibiotica Netropsin, Distamycin und/oder Anthelvencin und/oder deren Analoga, jeweils bevorzugt in HEPES-Puffer, eingesetzt. Nach einer bevorzugten Ausführungsform wird das gewählte Pyrrolamidin-Antibioticum ferner in Form eines Salzes, beispielsweise des Hydrochlorids, verwendet.

Das erfindungsgemäße Verfahren zum Nachweis von Fibrinmonomeren in Blut, insbesondere im menschlichen Blut, beruht darauf, daß man menschliches Venenblut in an sich bekannter Weise mit einem Anticoagulans, insbesondere Citrat, mischt, anschließend zentrifugiert und das Blutplasma zur Präzipitation mit der oben genannten erfindungsgemäßen Testkombination in Form einer Lösung versetzt. Anschließend wird das Produkt der Fällungsreaktion, d.h. das am Boden sedimentierte Präzipitat, in an sich bekannter Weise ausgewertet. Das mit dem Fällungsmittel bzw. der Testkombination versehene Plasma wird zur Auswertung vorzugsweise bei Raumtemperatur stehengelassen und anschließend zentrifugiert.

Die Vorteile der Erfindungskonzeption bestehen in der hohen diagnostischen Sicherheit sowie in der Verwendung eines kostengünstigen Fällungsmittels, besonders von Netropsin-hydrochlorid.

Die Erfindung wird im folgenden anhand von zwei Ausführungsbeispielen zum Nachweis von Fibrinmonomeren näher erläutert.

### Beispiel 1

Venenblut wird genau nach den für Gerinungsuntersuchungen geltenden Regeln gewonnen und daraus Citratplasma nach üblichen Methoden bereitgestellt.

Netropsin-HCl wird je nach Bedarf in einer Konzentration von 15 mg Netropsin/ml mit HEPES-Puffer (0, 15 M, pH 7,5) gelöst (Testkombination Netropsin RL).

0,4 ml Citratplasma werden mit 0,1 ml Netropsin-RL in einem Zentrifugenglas gemischt und 30 min bei Raumtemperatur stehengelassen. Dann wird die Probe 5 min bei einer relativen Zentrifugalbeschleunigung (RZB) von höchstens 50 zentrifugiert. Die am Boden sedimentierten Präzipitate werden nach folgenden Kriterien im - schräg einfallenden Licht beurteilt:

Flockige lockere Präzipitation: +

faserige Ausfällungen : + +

kompakte Niederschläge : + + +.

Die auf diesem Weg als negativ beurteilten Proben werden in schwarze Blockschälchen überführt und erneut im schräg einfallenden Licht beurteilt.

Zu jeder Meßreihe wird eine positive Kontrolle zum Vergleich mitgeführt.

## Beispiel 2

Vorbereitung, Durchführung und Auswertung der Fällung werden wie in Beispiel 1 vorgenommen. Als Fällungsmittel wird jedoch Distamycin HCl, das in einer Konzentration von 6 mg/ml im HEPES-Puffer gelöst wird, eingesetzt (Testkombination Distamycin RL).

Die Empfindlichkeit der Präzipitation beträgt, verglichen mit Netropsin, ca. 50%.

Unter Verwendung der erfindungsgemäßen Testkombination Netropsin RL wurden nach dem erfindungsgemäßen Verfahren in klinischen Labors bei Serienversuchen die nachstehenden Ergebnisse erzielt:

## Prüfung des erfindungsgemäßen Testverfahrens auf seine analytische Verwendbarkeit

-In vitro hergestelltes fibrinmonomerhaltiges Plasma zeigt eindeutig eine positive Reaktion.

-Von in Einzelpräparation gewonnenen Spaltprodukten des Fibrinogens und Fibrins (e-FDP, 1-FDP, e-fdp, 1-fdp) wird nur die Fraktion der frühen Fibrinspaltprodukte (e-fdp) erfaßt. Da e-fdp ebenfalls Ergebnisse einer thrombinbedingten Fibrinogenkonversion sind, wird dadurch die Spezifität des Tests nicht berührt. Ab einer Konzentration der niedermolekularen Spaltprodukte Fragment D (0,3 g/l) und Fragment E (0,15 g/l) wird eine Abschwächung der Präzipitation beobachtet.

-Eine fibrinogenabhängige Beeinflussung der Testergebnisse konnte bis zu einer Fibrinogenkonzentration im Plasma von 19 g/l nicht gefunden werden.

-Ein Einfluß von Heparin auf die Präzipitation konnte nicht festgestellt werden.

-Es besteht keine Abhängigkeit der Präzipitation vom Thrombocytengehalt des eingesetzten Plasmas.

-Es wurde kein Einfluß von Plasmaexpandern auf Dextran- und Gelatinebasis festgestellt.

-Der Test zeigt keine Beeinflussung durch Veränderung der Albumin-α-Globulin-, Immunglobulin-G-und der Gesamteiweißkonzentration des Plasmas.

## Untersuchungen zur Spezifität des erfindungsgemäßen Testverfahrens

Die Untersuchung von 40 haemostaseologisch gesunden hospitalisierten Patienten ergab unter Anwendung der für Gerinnungsuntersuchungen üblichen Blutabnahmetechniken in allen Fällen ein negatives Testergebnis (Spezifität = 1,0).

Untersuchung der Sensitivität des Testverfahrens

Es wurden 40 Patienten mit klinisch und labordiagnostisch gesicherter disseminierter intravasaler Gerinnung mit dem beschriebenen Test untersucht, der in 37 Fällen ein positives Ergebnis brachte. Damit erwies sich das Verfahren mit einer Sensitivität von 0.93 als labormäßig für die Diagnose der disseminierten intravasalen Gerinnung geeignet.

## Ansprüche

1. Verfahren zum Nachweis von Fibrinmonomeren in Blut durch
Mischen von Venenblut mit einem Anticoagulans, Zentrifugieren und Gewinnung des entsprechenden Blutplasmas aus dem Überstand,
Präzipitation mit einem Fällungsmittel sowie
Auswertung in üblicher Weise,
**gekennzeichnet durch**
Verwendung eines Antibioticums der Pyrrolamidin-Reihe als Fällungsmittel.

2. Verfahren nach Anspruch 1, gekennzeichnet durch Verwendung eines Antibioticums der Pyrrolamidin-Reihe in Form eines Salzes.

3. Verfahren nach Anspruch 1 oder 2, gekennzeichnet durch Verwendung des Antibioticums der Pyrrolamidin-Reihe in einer Pufferlösung.

4. Verfahren nach Anspruch 3, gekennzeichnet durch eine HEPES-Lösung als Pufferlösung.

5. Verfahren nach einem der Ansprüche 1 bis 4, gekennzeichnet durch Verwendung von Netropsin, Distamycin und/oder Anthelvencin und/oder Analoga dieser Antibiotica als Fällungsmittel.

6. Verfahren nach einem der Ansprüche 1 bis 5, gekennzeichnet durch Stehenlassen des mit dem Fällungsmittel versetzten Blutplasmas bei Raumtemperatur und anschließendes Zentrifugieren.

7. Verfahren nach einem der Ansprüche 1 bis 6, gekennzeichnet durch Verwendung von Citrat als Anticoagulans.

8. Verfahren nach einem der Ansprüche 1 bis 7, gekennzeichnet durch Verwendung von Netropsin-hydrochlorid und/ oder Distamcyin-hydrochlorid als Fällungsmittel.

9. Testkombination zur Durchführung des Verfahrens nach einem der Ansprüche 1 bis 8 auf der Basis eines Fällungsmittels für Fibrinmonomere, gekennzeichnet durch ein Antibioticum der Pyrrolamidin-Reihe als Fällungsmittel.

10. Testkombination nach Anspruch 9, gekennzeichnet durch ein Antibioticum der Pyrrolamidin-Reihe in Form eines Salzes als Fällungsmittel.

11. Testkombination nach Anspruch 9 oder 10, gekennzeichnet durch einen Puffer, in dem das Antibioticum der Pyrrolamidin-Reihe vorliegt.

12. Testkombination nach Anspruch 11, gekennzeichnet durch HEPES-Puffer.

13. Testkombination nach einem der Ansprüche 9 bis 12, gekennzeichnet durch Netropsin, Distamycin und/oder Anthelvencin und/oder Analoga dieser Antibiotica als Fällungsmittel.